# EUROPEAN PATENT APPLICATION

(11) **EP 1 843 156 A2**
(43) Date of publication of application: **10.10.2007**
(21) Application number: 07105613.9
(22) Date of filing: 04.04.2007
(51) Int. Cl.: G01N 33/49, G01N 33/52, B01D 39/14

(54) **Filter for separating blood cells**

(30) Priority: 04.04.2006 JP 2006103002; 30.03.2007 JP 2007094376
(71) Applicant: Fujifilm Corporation, Minato-ku Tokyo (JP)
(72) Inventor: Sakaino, Yoshiki, Ashigarakami-gun Kanagawa (JP); Yang, Bo, Ashigarakami-gun Kanagawa (JP); Ogawa, Shotaro, Fujinomiya-shi Shizuoka (JP)
(74) Representative: Banzer, Hans-Jörg

(57) **Abstract**

A filter for separating blood cells, which comprises: a substrate; and at least one water-insoluble substance fixed to the substrate, wherein the at least one water-insoluble substance has an equivalent circle diameter of 4 µm or less and a height equal to or larger than the equivalent circle diameter, wherein blood cells are separated by substantially being captured with the at least one water-insoluble substance; and a blood filtration instrument and a blood analytical device, using the filter for filtration of blood and body fluid.

## Description

### Background of the Invention

### 1. Field of the Invention

The present invention relates to a filter for separating blood cells, used in a blood test method of human being and other animals, and a filtering instrument and an analytical device, using the filter.

### 2. Description of the Related Art

A method of diagnosing disease of human being using blood, urine and the like as an analyte has conventionally been conducted for a long time as a method that can diagnose simply without damaging human body.

In particular, the blood enables many examination items to diagnose.

A wet chemistry analytical method has conventionally been developed as an analytical method for examining such many items. This is a method using a solution reagent. An apparatus using the wet chemistry analytical method for the purpose of examination of many items is generally complicated because of combining many reagent solutions corresponding to many items and handling of the apparatus and procedure are not simple.

To the above situation, a method that can easily conduct analysis is sought.

As one method, a so-called dry chemistry analytical method in which reagents required to detect specific components are contained in a dry state is being developed (JP-T-2001-512826 (the term "JP-T" as used herein means a published Japanese translation of a PCT patent application)).

However, even in a wet chemistry and a dry chemistry, where an analyte is blood, whole blood is not generally used, and blood cell component is removed from blood to provide for analysis in a form of blood plasma or blood serum. As a method for removing blood cell component, blood cell separation is hereto conducted with a method of using centrifugal force, thus requiring centrifugal separation work. When blood plasma after centrifugal separation is used as an analyte, it is necessary to once stop centrifugal operation and then supply blood plasma after the centrifugal separation. Thus, it is difficult to conduct blood plasma separation and detection in a series of operations, and there was the problem that time reaching detection is long.

To this problem, an apparatus for separating blood cells with a method of using a filter is developed (JP-A-10-227788 and the like). Although time required for blood cell separation is shortened to some extent, but removal of blood cells from whole blood (so-called "blood cell separation") and detection are separate works, and it cannot say that shortening of time is sufficient.

Further, where glass fibers are used as a filter for separating blood cells, there is the possibility that components eluted from glass fibers or components adsorbed on glass fibers affect the subsequent analysis of blood test. To this problem, a method of previously treating glass fibers with an organic acid such as acetic acid is proposed (JP-A-10-227788).

FUJI DRI-CHEM PLASMA FILTER PF (trade name, a product of Fujifilm Medical Co., Ltd.) is an instrument for separating blood cells in methods of using a filter (JP-A-10-227788 and the like), and filter glass fibers are filled in a cartridge made of a resin. The glass fibers are filled in a cartridge by ultrasonic fusing of cartridge, but the glass fibers are not directly fixed to the cartridge. Therefore, thickness of glass fibers changes by lots of glass fibers, and this gives rise to the following problems. When glass fibers having a thickness smaller than the general thickness are filled in a cartridge, a space is formed between the glass fibers and the cartridge, thereby blood cells pass through without passing through a glass fiber filter. On the other hand, when glass fibers having a thickness larger than the general thickness are filled in a cartridge, glass fibers become too dense, and as a result, blood cells cause clogging and filtration cannot be performed.

Additionally, a columnar substance fixed to a substrate called a pillar is prepared using micro/nano processing technology that is recently developed, and is attempted to apply to medical field (JP-A-2004-170935 and JP-A-2004-045358). For example, JP-A-2004-170935 prepares a pillar having a diameter of from 10 nm to 500 µm, a height of from 50 nm to 5,000 µm and an aspect ratio of 4 or more, but such a pillar is not used for the purpose of separating and recovering components in blood. Further, JP-A-2004-045358 attempts to separate components such as protein by utilizing resistance when a substance passes through a space of pillars, but does not refer to a method for separating by capturing cells with the pillar itself.

On the other hand, in an aging society, a blood test that can readily measure health condition is more and more increasing its importance, and is the means that can easily know change in the state of disease in lifestyle-related diseases. It is necessary in elderly persons and litestyle-xelated diseases to observe stages of progress of health condition/diseases with the passage of time, and occasions requiring a blood test are increasing. For this reason, a method is desired that not only medical personnel, but patients can collect blood by themselves and simply and quickly analyze the same.

To this desire, an analyzer in which blood collection by a needle, blood cell separation by filtration and centrifuge and wet chemistry analysis method by an electrode method are combined to integrate the means from blood collection to analysis is proposed (JP-A-2001-258868). However, this analyzer does not yet reach to sufficiently satisfy ease of operation. Further, there is the case that scattering of measurement values occurs, and the analyzer does not reach to be satisfactory from the standpoint of measurement precision in clinical test.

In medical sites, it is required that operations from collection of an analyte to analysis and detection thereof are further quickly conducted. In recent years, in-hospital infection becomes large social problem, and particularly defense of infection from blood is desired. As a blood test unit that can prevent men in charge of test from contacting with blood plasma or blood serum during the test, an analyzer that is combined with a light detector to integrate the means from blood collection to analysis is proposed (JP-A-2003-287533).

### Summary of the Invention

In the blood cell separation apparatus using the above-described glass fibers described above, there was the technical problem that it is difficult to fill sheet-like glass fibers having elasticity in a microstructural portion such as a microchip.

Sheet-like glass fibers (glass fiber filter) having elastic force and restorative force have the properties that cells such as red blood cells are liable to clog when a space volume (generally called void) among the glass fibers becomes small. When a glass fiber filter as a filtering material is filled in a microstructural portion of a microchip, it gives priority to fill the same such that a liquid filtered does not leak, and therefore, it is difficult to fill the glass fiber filter such that the voids do not change. As a result, the glass fiber filter collapses, and voids are liable to become small.

Consequently, where glass fibers as a filtering material are filled in a microstructural portion of a microchip, there was the disadvantage that red blood cells clog in the filtering material, and a sufficient amount of blood plasma cannot be recovered.

Contrary to this, where glass fibers are airily filled such that the glass fibers do not collapse, there was the problem that a space is generated between a cartridge member filling glass fibers, and the glass fibers, and as a result, blood cells pass thought without being captured. Therefore, a method that maintains voids of glass fibers constant, and can fill the glass fibers without generation of spaces between the cartridge member and the glass fibers was required.

Accordingly, one object of the present invention is to provide a filter for filtration of a liquid that uses a water-insoluble substance having a diameter similar to that of glass fibers and comprises a substrate and the water-insoluble substance, at least one portion thereof being fixed to the substrate, as a filter for separating cells such as blood cells.

Another object of the invention is to provide a blood filtering instrument that can recover by filtration, blood plasma having the same component as that by centrifugal separation in a short period of time by using the filter.

As a result of extensive and intensive investigations, the present inventors have found that the above objects can be achieved by, for example, a filter using a water-insoluble substance having an equivalent circle diameter of 4 µm or more and a height equal to or larger than the equivalent circle diameter, the water-insoluble substance being fixed to a substrate.

That is, the present has achieved the above objects by the following constitutions.
(1) A filter for separating blood cells, which comprises:
   a substrate; and
   at least one water-insoluble substance fixed to the substrate, wherein the at least one water-insoluble substance has an equivalent circle diameter of 4 µm or less and a height equal to or larger than the equivalent circle diameter,
   wherein blood cells are separated by substantially being captured with the at least one water-insoluble substance.
(2) The filter for separating blood cells as described in (1) above,
   wherein the at least one water-insoluble substance comprises a first water-insoluble substance and a second water-insoluble substance, and
   a distance between the first and second water-insoluble substances is 4 µm or more.
(3) The filter for separating blood cells as described in (1) or (2) above,
   wherein the at least one water-insoluble substance is a plurality of water-insoluble substances, and
   the plurality of water-insoluble substances are arranged in a plurality of rows at even intervals in each row.
(4) The filter for separating blood cells as described in (3) above,
   wherein the interval in each row is 4 µm or more.
(5) The filter for separating blood cells as described in (3) or (4) above,
   wherein the plurality of water-insoluble substances are arranged in a checkered pattern.
(6) The filter for separating blood cells as described in any of (3) to (5) above,
   wherein the plurality of water-insoluble substances are pillars.
(7) The filter for separating blood cells as described in any of (1) to (6) above, which has an inlet portion and an outlet portion,
   wherein the at least one water-insoluble substance is a plurality of water-insoluble substances, and
   the plurality of water-insoluble substances are arranged from the inlet portion to the outlet portion in a plurality of rows at even intervals in each row, and
   wherein an interval in the inlet portion and an interval in the outlet portion are substantially 1.5 times or more different.
(8) A blood filtering instrument, which comprises a filter for separating blood cells as described in any of (1) to (7) above.
(9) A method for separating blood cells, which comprises utilizing a filter for separating blood cells as described in any of (1) to (7) above.
(10) A blood pre-treatment element, which comprises a filter for separating blood cells as described in any of (1) to (7) above.
(11) An analytical element, which comprises a filter for separating blood cells as described in any of (1) to (7) above.

### Brief Description of the Drawings

Fig. 1 is a plain view showing the arrangement of resin-made pillars,
Fig. 2 is a figure of scanning electron micrograph of resin-made pillars prepared;
Fig. 3 is a figure of photograph of blood cell separation with a blood cell separation filter using resin-made pillars;
Fig. 4 is a figure of photograph of component that blood cells were separated from whole blood with a blood cell separation filter;
Fig. 5 is state that blood cells are entangled with pillars when a whole blood sample liquid is flown in a custom chip having pillars arranged therein;
Fig. 6 is state that blood cells are entangled with pillars having a diameter of 3 µm when whole blood sample liquid is flown in a custom chip having the pillars arranged therein observed by a high speed camera;
Fig. 7 is a frame format view showing one exemplary embodiment of a multi-measuring dry analytical device;
Fig- 8 is a frame format view showing one exemplary embodiment of a multi-measuring dry analytical device (after fabrication);
Fig. 9 is a frame format view showing one exemplary embodiment of a blood collection unit;
Fig. 10 is a frame format view showing one exemplary embodiment of a blood collection unit (when collecting blood) ;
Fig. 11 is a frame format view showing a measuring apparatus;
Fig. 12 is a cross sectional view showing one exemplary embodiment of a fitting type dry analytical device;
Fig. 13A is a top view showing an upper member 30 of the fitting type dry analytical device, and Fig. 13B is a cross sectional view showing an upper member 30 thereof;
Fig. 14A is a top view showing a lower member 40 of the fitting type dry analytical device, and Fig. 14B is a cross sectional view showing a lower member 40 thereof;
Fig. 15 is a view showing an exemplary example of a blood filtration instrument and a filter receiving member;

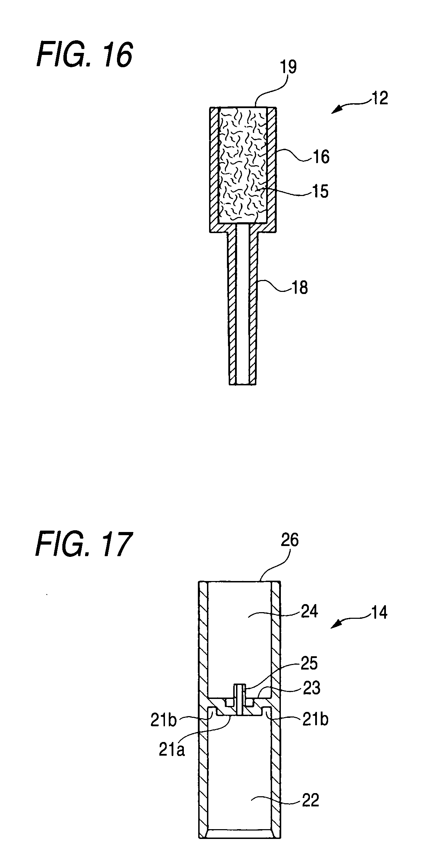

Fig. 16 is a view showing an exemplary example of a filter receiving member; and
Fig. 17 is a view showing an exemplary example of a holder member,
wherein A100 denotes multi-measuring dry analytical element; A1 denotes flow passage; A2 denotes part supporting a development reaction reagent; A3 denotes injection port; A4 denotes upper lid; A5 denotes lower material; A6 denotes filtering material; A7 denotes development reaction reagent; E1 denotes bonding direction of an upper lid; E2 denotes arrow showing arrangement place of a filtering material; E3 denotes arrow showing arrangement place of a development reaction reagent; B100 denotes blood collection unit; B1 denotes blood collection instrument; B2 denotes puncture needle; Cl denotes mounting direction of a multi-measuring dry analytical element; C2 denotes sliding direction when reducing pressure; D denotes whole blood; 10 denotes liquid filtration instrument; 12 denotes filter member; 14 denotes holder member; 15, 36 each denotes liquid filtration filter; 17, 52 each denotes sealing member (porous membrane) ; 19 denotes opening at liquid outlet side (upper side) of a filter member; 28, 55 each denotes fitting portion; 30 denotes upper member; 40 denotes lower member; 50 denotes dry analytical device; 54 denotes dry analytical element; 100 denotes measuring apparatus; 71 denotes multi-measuring dry analytical element installation part; 72 denotes light source; 73 denotes light-variable part; 74 denotes wavelength-variable part; 75a, 75b, 75c denote lenses; 76 denotes area sensor; and 77 denotes computer.

### Detailed Description of the Invention

The invention is described in detail below.

### Filter for Filtration of Liquid

The filter for filtration of a liquid of the invention is a filter for filtration of a liquid, for recovering a filtrate after passing a liquid such as body fluid and blood through a filter and filtering off.

### Water-Insoluble Substance

Examples of material of the water-insoluble substance preferably include fibrous materials such as resin fibers and glass fibers, and columnar materials such as so-called pillars made of a resin, a metal or silicon.

Examples of material of the resin-made fibers include fibers comprising resins such as polyester, polyethylene, polypropylene, nylon, cellulose and cellulose acetate, and fibers comprising composite materials such as polyester/nylon and polyester/cellulose.

Examples of material of the glass fibers include soda glass, low-alkali glass, borosilicate glass and quartz.

Examples of material of the resin-made pillars include pillars comprising resins such as polyester, polyethylene, polypropylene, nylon, cellulose and cellulose acetate, and pillars comprising composite materials such as polyester/nylon and polyester/cellulose.

Examples of material of the metal-made pillars include metals such as iron, copper, silver, gold, platinum, nickel, stainless steel and titanium, and metal oxides such as zirconium oxide and titanium oxide.

Example of material of the silicon-made pillars includes silicon wafer.

Filtration is preferably conducted using a water-insoluble substance having an equivalent circle diameter of 4 µm or less as the water-insoluble substance. Considering mechanical strength of the water-insoluble substance, the diameter is preferably from 0.4 to 4 µm, and more preferably from 0.4 to 2 µm.

The term "equivalent circle diameter" as used herein means a so-called equivalent diameter, and is a term generally used in the filed of mechanical engineering. Assuming a circular tube equivalent to a tube having an optional cross section (in the invention, corresponding to water-insoluble substance, fibers and glass fibers), a diameter of the equivalent circular tube is called an equivalent diameter, and the equivalent diameter (d_{eq}) is defined by d_{eq}=4A/p wherein A is a cross section of a tube and p is wetted circumferential length (perimeter). When this definition is applied to a circular tube, its equivalent diameter consists with a diameter of the circular tube. The equivalent diameter is used to estimate flow or heat transfer properties of its tube on the basis of the data of the equivalent circular tube, and represents a space scale (representative length) of phenomenon. The equivalent diameter is d_{eq}=4a²/4a=a in a regular tetragon tube having one side length of "a", and d_{eq}=2h in a flow between parallel flat plates having a passage height of h. Details of those are described, in Encyclopedia of Mechanical Engineer (edition by The Japan Society of Mechanical Engineers, 1997, Maruzen Co., Ltd.).

An equivalent circle radius is calculated in the same manner as in the equivalent circle diameter as described above.

The water-insoluble substance used has a height equal to or larger than the equivalent circle diameter. The ratio of the height to the equivalent circle diameter is preferably 2 or more, and more preferably 10 or more.

For example, where the water-insoluble substance is a fibrous material, strictly a height of the water-insoluble substance is a length of fiber, and a diameter of fiber is the equivalent circle diameter. However, as a matter of convenience, it may be considered that the maximum height of the whole water-insoluble substance fixed to a substrate is a height of the water-insoluble substance.

It verifies here that the water-insoluble substance is capable of capturing blood cells.

It was observed as to how red blood cells in whole blood are captured with a glass fiber filter used as a filtering material for filtration of blood. Whole blood was collected from a healthy man with a vacuum collection tube using heparin lithium as an anticoagulant. Hct value in this case was 45%. The whole blood was added dropwise in an amount of 10 µl to a glass fiber filter GF/D (diameter of glass fiber: about 3 µm or less), a product of Whatman Co., at room temperature. The glass fiber filter having the whole blood added dropwise thereto was quickly introduced into a 0.1 mol/liter phosphoric acid buffer solution (pH 7.4) containing 1% glutaraldehyde, and allowed to stand at room temperature for 2 hours to harden blood cells. Subsequently, the glass fiber filter was dipped in a water/t-butanol mixed liquid, and a water/t-butanol mixing ratio was gradually varied, ultimately substituting with t-butanol, followed by allowing the solution to stand in a refrigerator to freeze. The frozen t-butanol solution containing the glass fiber filter was placed in a freeze dryer to remove a solvent. The thus-obtained glass fiber filter in a dry state to which the whole blood had been added dropwise was observed with a scanning electron microscope. As a result, it was confirmed that red blood cell could be captured with the glass fibers having diameter of 3 µm or less.

For the sake of comparison, the same experiments as above were conducting using glass fiber filters having a diameter of about 8 µm and 10 µm, and an acetyl cellulose fiber filter having a diameter of about 5 µm, as a filtering material. The glass fibers having a diameter of about 8 µm do not capture blood cells. The glass fiber filters having a diameter of about 10 µm and the acetyl cellulose fibers having a diameter of about 15 µm could not capture blood cells at all.

From the above, it is seen that where whole blood is used as an analyte, red blood cells can quickly and efficiently be removed from the whole blood by using fibers having a specific equivalent circle diameter, i.e., a water-insoluble substance, as a filtering material for filtration of blood. Further, it is not necessary to operate a specific apparatus to remove red blood cells from whole blood. As a result, blood plasma can quickly be supplied to a reagent, and time reaching measurement can be shortened.

It was confirmed the case that red blood cells are entangled with glass fibers, and the glass fibers can capture red blood cells. It is unclear by what force red blood cells are entangled with the glass fibers. However, even thought it is a so-called mechanical capture or a chemical adsorption, if a state that red blood cells are entangled with a water-insoluble substance such as glass fibers and adhered thereto can be formed, it can say that the water-insoluble substance could capture red blood cells.

Further, it is as a matter of course that glass fibers continue to capture red blood cells, and even though red blood cells are instantaneously captured with glass fibers, blood plasma and blood serum that are a liquid around red blood cells continue to flow around the glass fibers. Therefore, flow rate of red blood cells is relatively slower than flow rate of blood plasma and blood serum, and as a result, components of blood plasma and blood serum can be recovered by filtration. Thus, when the glass fiber filter is used as a filtering material, red blood cells are substantially captured with glass fibers, thereby achieving to recover blood plasma and blood serum and separate blood cells from a liquid. Similarly, cells such as white blood cells and blood platelets are instantaneously adsorbed on or captured with glass fibers, and flow rate of those is slower than flow rate of blood plasma and blood serum, thereby achieving separation of white blood cells and blood platelets from whole blood.

Further, it is possible to recover components in cells by capturing cells such as red blood cells with a filter, treating the filter with a solution containing a protein-degrading enzyme such as protease and a surfactant such as sodium dodecyl sulfate (SDS) to solubilize the cells and then washing the filter with 10 mmol/liter tris·bishydroxymethyl aminoethane (generally called Tris) buffer having pH of about 9 or a 70% ethanol aqueous solution.

### Substrate

In the filter for separating blood cells of the invention, plural water-insoluble substrates are fixed to a substrate. The substrate used is not particularly limited, and various materials such as materials generally used in blood treatment, for example, semiconductor materials such as glass, resin and silicon wafer, can be used.

### Fixing Embodiment and Production Method of Water-Insoluble Substance

In the filter for separating blood cells of the invention, the water-insoluble substance has at least one contact point to the substrate. For example, in the case of the above-described pillar, the pillar generally has one contact point fixed to the substrate.

In preparing the water-insoluble substance, its shape is not particularly limited as described above, and for example, fibrous shape or pillar shape can be used.

The water-insoluble substance of the invention can be prepared by, for example, molding a photocurable resin into a structure having a diameter of an equivalent circle diameter of 3.8 µm or less with a photo-shaping technique. In this case, by preparing a structure comprising mutually crosslinked structures each having an equivalent circle diameter of 3.8 µm or less, mechanical strength is imparted and as a result, a structure satisfying both filtration performance and mechanical strength can be prepared. Examples of the shape of such a structure include a structure comprising pillars mutually crosslinked, a structure comprising fibers mutually crosslinked, a mesh structure of curb shape, checkered pattern or honeycomb shape, and its crosslinked product.

A production method of the pillar may be a method of remaining silicon in a columnar shape by exposing and etching a silicon wafer, generally conducted in semiconductor processing, or may use an imprinting method of compression bonding a mold having a depressed shape to a resin, and peeling the mold, thereby forming columnar projections on the surface of the resin, as reported in micro-nano processing.

Examples of the water-insoluble substance having at least two contact points to the substrate include lattice-like or net-like steric structures by means of a steric shaping using a photo-crosslinking method. Further examples include substances having a steric structure, such as a micromesh filter, prepared using etching technology of MEMS (micro electro mechanical system) as reported in 16th International Conference on Micro Electro Mechanical Systems (MEMS-03), Kyoto, 223/226 (2003) by Prof. Shuichi Shoji, Waseda University. Further, the means for preparing a structure such that a pore size of a porous membrane such as polysulfone or acetyl cellulose is extremely increased so as to form a hole, so that columnar polymer remains in the membrane is also effective.

In the filter for separating blood cells of the invention, configuration of arranging the water-insoluble substances on the substrate is not particularly limited, but it is preferable to arrange the water-insoluble substances at even intervals in rows. For example, pillars having an equivalent circle diameter of 4 µm or less are arranged in curb shape, checkered pattern or honeycomb shape. Where it is a structure in a checkered pattern such that a second row is arranged in the same intervals as in the first row so as to deviate half cycle to the first row arranged in even intervals, it is considered that the chance that cells passed through pillars of the first row are caught and captured with pillars of the second row. Therefore, the arrangement of a checkered pattern is preferable.

Further, the interval between pillar and pillar is not particularly limited. Interval between pillars can be larger than a size through which cells can pass so far as clogging of cells occurs and the cells are captured. For example, where cells are red blood cells, the interval between pillar and pillar is preferably 4 µm or more, and more preferably from 6 to 40 µm. On the other hand, if it is desired to completely capture cells even if clogging of cells occurs, the interval between the pillars can be smaller than a size through which cells can pass. For example, where cells are red blood cells, the size is preferably less than 4 µm, and more preferably 0.5 µm or less. Therefore, the interval between pillar and pillar may stepwise or continuously be changed from an inlet side of the filtration filter to an outlet side thereof. For example, the interval between pillar and pillar at an inlet side of a filter is 20 µm, the pillar interval is stepwise narrowed toward the outlet side from the inlet side, and the pillar interval at the outlet side is 0.5 µm. By this configuration, function can be imparted that red blood cells in blood are stepwise captured, and finally, red blood cells do not leak at all. More preferably, the interval between pillar and pillar at an inlet side of the filtration filter is preferably 6 µm or more in order to prevent clogging of cells, and that at an outlet side is preferably 4 µm or less in order to capture the blood cells completely, so that the intervals in an inlet portion and an outlet portion are substantially 1.5 times or more different.

Further, the shape of the substrate having the water-insoluble substance fixed thereto (filter for separating blood cells) is not particularly limited, and may be any shape such as square, rectangle, triangle and circular form. When blood is flown to separate blood cells and recover blood plasma, in order to recover blood plasma from a slight amount of blood with good efficiency, a shape that is long in blood-flowing direction is preferable, and a rectangle is more preferable. Separation Method of Blood Cells

As described above, blood cells are captured with the water-insoluble substance. Therefore, blood cells can be separated and removed from blood by merely passing blood through the filter for separating blood cells of the invention. The means for passing blood through the filter of the invention is not limited. For example, structure such as a blood filtering instrument as described hereinafter can be employed.

### Analyte

An analyte to be provided to the filter for separating blood cells of the invention includes blood of human being and animals.

### Blood Filtering Instrument

The blood filtering instrument of the invention can be constituted as a blood filtering unit comprising a filter for separating blood cells comprising a substrate having fixed thereto a blood filtering material comprising a water-insoluble substance, and a constitutive member receiving the filter for separating blood cells, as described hereinafter.

A filter for separating blood cells used in a blood filtering unit that can be used as one example of the blood filtering instrument of the invention, a microporous membrane and a member constituting a circumference of the filter are described below.

In the blood filtering material, the filter for separating blood cells is arranged with a size having a length of from about 0.5 to 100 mm, a width of from about 0.5 to 50 mm and a thickness of from about 0.02 to 2 mm, and preferably a length of from about 5 to 30 mm, a width of from about 0.5 to 5 mm and a thickness of from about 0.05 to 0.2 mm, and used as a filtering site.

### Microporous Membrane

It is further effective to arrange a microporous membrane for further promoting separation of blood cells and blood plasma and inhibiting leakage of blood cells at the outlet side of a filtrate in a filtering site having the water-insoluble substance provided thereon.

The microporous membrane has a hydrophilicized surface and has blood cell separability, and therefore specifically separates blood cells and blood plasma from whole blood without hemolyzing in an extent of substantially giving influence to analytical value. Pore size of the microporous membrane is preferably smaller than voids of the water-insoluble substance, and the minimum pore size is 0.1 µm or more, preferably from about 0.3 to 5 µm, and more preferably from about 0.5 to 2 µm. The porosity is preferable as being high. Specifically, the porosity is in a range of from about 40 to about 95%, preferably from about 50 to about 95%, and more preferably from about 70 to about 95%. Examples of the microporous membrane include a polysulfone membrane, a flurine-containing polymer membrane, a cellulose acetate membrane and a nitrocellulose membrane. Further examples include microporous membranes having a surface hydrophilicized by hydrolysis, a hydrophilic polymer, a surfactant or the like.

Preferable microporous membranes are a polysulfone membrane, a cellulose acetate membrane and the like, and particularly preferable microporous membrane is a polysulfone membrane. In the blood filtering material, the water-insoluble substance is provided at a blood supply side, and the microporous membrane is provided at an outlet side.

The microporous membrane has a thickness of from about 0.05 to 0.7 mm, and preferably from about 0.1 to 0.3 mm, and one microporous membrane is generally used. However, if desired and necessary, plural microporous membranes can be used.

### Member Constituting Circumference of Filter for Separating Blood Cells

A member constituting circumference of the filter receives the filter for separating blood cells comprising a substrate having fixed thereto the blood filtering material, and is provided with a blood inlet and a filtrate outlet. This constitutive member is generally prepared in an embodiment such that a main body receiving the filter and a lid thereof are separated. In general, at least one opening is provided on each of those, and one opening is used as a blood supply port, and as the case may be, additionally as a pressure port, and other opening is used as a suction port, and as the case may be, additionally as a discharge port of blood plasma or blood serum further filtered. Discharge ports of the filtered blood plasma and blood serum can be provided separately. When the constitutive member is a quadrangle and a lid is provided at a side face thereof, both the blood supply port and the suction port can be provided on the main body.

It is necessary to design volume of a storing part of the filter for separating blood cells in consideration of the total volume when the filter to be stored is in a dried state and the filter absorbs blood and is swelled. Where the volume of the storing part is too small to the total volume of the filter, filtration may not proceed with good efficiency, or hemolysis may occur. On the other hand, the volume of the storing part is too large to the total volume of the filter, there is the possibility that the substance to be filtered is not filtered, and passes through the filter. The proportion of the volume of the storing part to the total volume of the filtering material when dried is generally from 50 to 300%, preferably from 70 to 200%, and more preferably from 90 to 150%, although varying depending on the degree of swelling of the filter.

Further, it is necessary that the filter and a side wall surface of the storing part are closely contacted, and it is also necessary to be constructed such that flow passage not passing through the filter when sucking whole blood is not formed.

Amount of the blood filtering material fixed to the substrate varies depending on the density of the water-insoluble substance used as a filtering material. However, generally, when a pillar substance is used as the water-insoluble substance, it is desirable that the amount is from 1/1, 000 to 100 times of the number of red blood cells per volume of blood filtered. The number of red blood cells present in blood of human being is from 4,300,000 to 5,800,000 per 1 µl of whole blood in healthy men. Therefore, specifically it is desirable to design such that the number of the pillar substance is preferably from 4,000 to 400,000,000, and more preferably about 400, 000, per 1 µl of blood used for filtration.

A filtrate receiving tank that receives blood plasma filtered can be provided at an outlet side of the filtrate in the member constituting the circumference of the filter for separating blood cells. The receiving tank is preferably designed from design of an analyzer such that the analyzer can suck blood plasma from at least a central direction of the constitutive member, and as a result, the filtrate outlet in the blood filtering material receiving chamber and a passage of the filtrate to the receiving tank are provided deviating from the center of the constitutive member.

Volume of the filtrate receiving tank is preferably from 0.05 µl to 1 ml.

Material constituting the circumference of the filter for separating blood cells is preferably a thermoplastic or thermosetting plastic. For example, transparent or opaque resins such as high impact polystyrene, methacrylic acid ester, polyethylene, polypropylene, polyester, nylon, polycarbonate and various copolymers are used.

A mounting method of the main body and lid is generally by bonding using an adhesive, fusion and the like.

Shape of the filter is not particularly limited. Where a large amount of blood is filtered, circular shape or polygonal shape is preferably from the point of ease of production thereof. In this case, the filter is designed to have the same size as an internal cross section of the member main body constituting the circumference of the filter for separating blood cells, thereby it is possible to prevent leakage of blood plasma from a side of the filter. Further, when the filter has a quadrangular shape, cut loss of the filter prepared is minimized, which is preferable.

Use method of the blood filtering instrument is that blood is supplied from a blood inlet of the blood filtering instrument, and blood plasma or blood serum as a filtrate is collected from an opening at an opposite side thereof. Amount of blood supplied is from about 1. 2 to 5 times, and preferably from about 2 to 4 times, the volume of the filter. In filtering, pressure is applied from the blood inlet side or pressure is reduced from the opposite side, to promote filtration. A method of utilizing peristal or syringe is simple as the pressure application or pressure reduction means. Distance moving a piston of the syringe is that moving volume of the piston is about 2 to 5 times the volume of the filter. Moving speed is from about 1 to 500 ml/min, and preferably from about 20 to 100 ml/min, per 1 cm². Filtering instrument after use is generally disposable.

Blood plasma or blood serum obtained by filtration is analyzed according to the conventional method. The filtering unit is effective particularly in the case of analyzing multiple items using a dry analytical device.

The blood filtering unit is disclosed in, for example, JP-A-9-196911, JP-A-9-276631, JP-A-9-297133, JP-A-10-225448 and JP-A-10-227788.

### Analytical Element

Multi-measuring dry analytical device that can be used as one example of the blood analytical element (blood analytical device such as multi-measuring dry analytical device) of the invention is described below.

The multi-measuring dry analytical device uses an area sensor, a line sensor or an electrochemical detector as a detector. Therefore, the detector is described below.

### Detector

(a) The area sensor can use any sensors so far as it is arranged so as to be able to detect light such as ultraviolet light, visible light and infrared light, or electromagnetic wave and obtain two-dimensional information. Examples of the area sensor include CCD, MOS and photographic films. Of those, CCD is preferable. By detecting the multi-measuring dry analytical device using the area sensor, measurement result can be obtained from information of 1,000 pixels or more regarding one item, and simultaneous measurement of multiple items is possible.
(b) The line sensor can use any sensors so far as it is arranged so as to be able to detect light such as ultraviolet light, visible light and infrared light, or electromagnetic wave and obtain one-dimensional information. Examples of the line sensor include photodiode array (PDA) and photographic films arranged so as to detect light in slit shape. Of those, photodiode array is preferable. By detecting the above-described specific multi-measuring dry analytical device using the line sensor, simultaneous measurement of multiple items is possible.
(c) The electrochemical detector can use detector so far as it can measure amount of the current, potential difference, electric conductivity and resistance. Examples of the electrochemical detector include electrodes of a conductive substance alone, such as a gold electrode, a platinum electrode, a silver electrode and a carbon electrode; a silver-silver chloride electrode, an oxygen electrode, composite electrodes such as modified electrode covered with an enzyme such as glucose oxidase, and their combinations. Of those, the modified electrode covered with covered with an enzyme such as glucose oxidase is preferable. By detecting the above-described specific multi-measuring dry analytical device using the electrochemical sensor, simultaneous measurement of multiple items is possible.

The filter for separating blood cells of the invention is used as filtering material for filtration of blood in an analytical device such as the above-described multi-measuring dry analytical device.

The multi-measuring dry analytical device is described in detail below. An embodiment using the area sensor (a) as a detector is described hereinafter. An embodiment using the line sensor (b) as a detector and an embodiment using the electrochemical detector (c) as a detector can apply similar to the embodiment of the area sensor (a).

The multi-measuring dry analytical device has a flow passage, a (development) reaction reagent and a part supporting the (development) reaction reagent (The term "dry analytical element" used herein means, for example, a (development) reaction reagent which reacts (and develops) by contacting with a filtrate (blood plasma) passed through a water-insoluble substance filter, and a part supporting the same. This may be representatively described by the part supporting the (development) reaction reagent.). It is preferable that at least one of width, depth and length of the flow passage is 1 mm or more, and width of the part supporting the (development) reaction reagent is 2 times or more the width of the flow passage, and/or length of the part supporting the (development) reaction reagent is 0.4 time or more the length of the flow passage.

The flow passage is described below.

As described above, the flow passage is that at least one of width, depth and length of the flow passage is preferably 1 mm or more, more preferably in a range of from 1 to 100 mm, and most preferably in a range of from 1 to 30 mm. In this range, an analyte proceeds in the flow passage efficiently, which is preferable.

The flow passage can be any embodiment so far as blood as an analyte can pass through the same.

Further, the flow passage may be one passage or may be branched into two or more passages. The flow passage can have any form of a linear form, curved form and the like, but a linear form is preferable.

Material of the flow passage can be any material so far as whole blood or blood plasma as an analyte can pass through the same efficiently. Examples of the material include resins such as rubbers and plastics, and silicon-containing substances.

Examples of the plastics or rubbers include polymethyl methacrylate (PMMA), polycyclic olefin (PCO), polycarbonate (PC), polystyrene (PS), polyethylene (PE), polyethylene terephthalate (PET), polypropylene (PP), polydimethylsiloxane (PDMS), natural rubber, synthetic rubber and their derivatives.

Examples of the silicon-containing substance include glass, quartz, amorphous silicon such as silicon wafer, and silicone such as polymethylsiloxane.

Of those, PMMA, PCO, PS, PC, glass and silicon wafer are preferable.

The flow passage can be formed on a solid substrate by fine processing technology. Examples of the material used include metal, silicon, Teflon, glass, ceramics, plastic and rubber.

Examples of the plastic include PCO, PS, PC, PMMA, PE, PET and PP. Examples of the rubber include natural rubber, synthetic rubber, silicon rubber and PDMS.

Examples of the silicon-containing substance include glass, quartz, amorphous silicon such as silicon wafer, and silicone such as polymethylsiloxane.

Particularly preferable examples include PMMA, PCO, PS, PC, PET, PDMS, glass and silicon wafer.

Fine processing technology for forming the flow passage includes the methods described in, for example, Microreactor, New-Generation Synthetic Technology (2003, published by CMC, supervised by Junichi Yoshida, Graduate School of Kyoto University, professor of engineering research) and Fine Processing Technology, Applied Edition, Application to Photonics, Electronics and Mechatronics (2003, published by NTS, edition of The Society of Polymer Science, Japan, Committee of Events).

Representative examples of the fine processing technology include LIGA technology using X ray lithography, high aspect ratio photolithography using EPON SU-8, microdischarge processing method (µ-EDM), silicon high aspect ratio processing method by Deep RIE, Hot Emboss processing method, photo-shaping method, laser processing method, ion beam processing method and mechanical micro-cutting processing method using a micro-tool made of a hard material such as diamond. Those technologies may be used alone or as combination of those. The preferable fine processing technologies are LIGA technology using X ray lithography, high aspect ratio photolithography using EPON SU-8, microdischarge processing method (µ-EDM), and mechanical micro-cutting processing method.

The flow passage can further be prepared by using a pattern formed by using a photoresist on a silicon wafer as a mold, pouring a resin in the mold, and solidifying the resin (molding method). The molding method can use a silicone resin represented by PDMS or its derivative.

It is desirable that according to need, the surface of the flow passage is treated or modified such that whole blood or blood plasma as an analyte can pass through the flow passage quickly. A surface treatment or modification method varies depending on a material constituting the flow passage, but the conventional method can be utilized. Examples of the method that can be used include plasma treatment, glow treatment, corona treatment, a method using a surface treating agent such as a silane coupling agent, and a surface treatment method using polyhydroxyethyl methacrylate (PHEMA), polymethoxyethyl acrylate (PMEA) or acrylic polymer.

The flow passage may be a part or the whole of the multi-measuring dry analytical device. That is, the flow passage can be prepared as a part or the whole of the multi-measuring dry analytical device by using a fine processing technology generally utilized to a so-called microreactor or a minute analytical element.

The preparation method of the microreactor and the minute analytical element can use the method as described in, for example, MICROREACTOR (supervised by Junichi Yoshida, published by CMC).

The (development) reaction reagent is described below.

The development reaction reagent is a reagent necessary for qualitative and quantitative analysis of components to be measured in an analyte, and means a reagent that reacts with the components to be measured in an analyte and colors, and a reagent that emits light by the action of light, electric or chemical reaction and the like, such as fluorescence and emission. In the invention, the reagent can appropriately be selected according to the kind of an analyte and the item to be measured. Examples of the reagent used include Fuji Dry Chem Mount Slide GLU-P (measurement wavelength: 505 nm, measurement component: glucose) andTBIL-P (measurement wavelength: 540 nm, measurement component: total bilirubin) , products of Fuji Photo Film Co., Ltd., now FUJIFILM Corporation). In the invention, the development reaction reagent contained in the multi-measuring dry analytical device uses a dried reagent. The dried reagent means a reagent used in so-called dry chemistry. Any reagent can be used so far as it is a reagent that can be used in dry chemistry.

Examples of the reagent used include reagents as described in, for example, Fuji Film Research Report No. 40 (Fuji Photo Film Co., Ltd,. 1995), page 83, and Clinical Pathology, extra addition, special topic No. 106, New Expansion of Dry Chemistry and Simple Test (The Clinical Pathology Press. 1997).

When the electrochemical detector is used as a detector, in place of a development reaction reagent, an enzyme electrode prepared by mixing a carbon paste comprising, for example, glucose oxidase (GOD), 1,1'-dimethylferrocene, and a mixture of a graphite powder and paraffin, and solidifying the mixture is used as an acting pole, a silver/silver chloride electrode is used as a reference pole, a platinum wire is used as a counter pole, and amount of the current increasing by glucose concentration in an analyte can be measured. More specifically, there are reagents described in, for example, Okuda, Mizutani, Yabuki, et al., Hokkaido Industrial Research Institute, Report No. 290, pages 173-177 (1991).

The part supporting the (development) reaction reagent is described below.

### Part Supporting (Development) Reaction Reagent (Dry Analytical Element)

An embodiment using the development reaction reagent is mainly described below. When the electrochemical detector is used as a detector, there is the same as in the part supporting the development reaction reagent in the embodiment using the area sensor, except that what the part supporting the reaction reagent supports is a reaction reagent.

As described above, the part supporting the development reaction reagent is preferably that its width is 2 times or more the width of the flow passage, and/or its length is 0.4 time or more the length of the flow passage.

The part supporting the development reaction reagent may be either only one, and two or more. When the part is two or more, those parts are collected in one portion, or may separately be arranged.

The part supporting the development reaction reagent may be in an embodiment connecting to the flow passage, or may be an embodiment that the part supporting the development reaction reagent is incorporated in the flow passage. In the embodiment of connecting to the flow passage, the part supporting the development reaction reagent may be a cell. The cell may have any embodiment so far as the width and/or length to the flow passage are satisfied with the above-described requirements. Materials of the cell include the same materials as described in the flow passage. Further, the preferable materials are the same as in the flow passage.

Connection between the flow passage and the part supporting the development reaction reagent can use a bonding technology. General bonding technologies are roughly classified into solid phase bonding and liquid phase bonding. In the conventional bonding technologies, pressure bonding and diffusion bonding are the representative bonding method as the solid phase bonding, and welding, eutectic bonding, soldering and adhesion are the representative bonding method as the liquid phase bonding.

Further, highly precise bonding method that does not involve destruction of a minute structure such as a flow passage by modification or large deformation of a material due to high temperature heating is desirable, and examples of such a technology include silicon direct bonding, anode bonding, surface activation bonding, direct bonding using hydrogen bond, bonding using HF aqueous solution, Au-Si eutectic bonding and void-free adhesion.

Further, bonding using ultrasonic wave, laser or the like, and bonding using an adhesive, an adhesive tape or the like may be used, and bonding may be performed by only pressure.

The part supporting the development reaction reagent may support the reagent in any embodiment so far as it can support the development reaction reagent. Examples of the embodiment include a test paper, a disposable electrode, a magnetic material and an analytical film. When a film is used, it may be a single layer film or a multilayered film.

Preferably, a dry multilayered film is used as a reagent layer in the part supporting the development reaction reagent. The dry multilayered film can incorporate all or a part of reagents necessary for qualitative and quantitative analysis of components to be measured in an analyte, which is preferable. The dry multilayered film includes films used in the dry chemistry described before. Specifically, the examples of such a film include films as described in, for example, Fuji Film Research Report No. 40 (Fuji Photo Film Co., Ltd., 1995), page 83, and Clinical Pathology, extra addition, special topic No. 106, New Expansion of Dry Chemistry and Simple Test (The Clinical Pathology Press. 1997). When the dry multilayered film is used as the reagent layer in the part supporting the development reaction reagent, it is easy to conduct a multi-stage reaction stepwise, which is preferable. Additionally, the reagent layer having the same quality can be prepared in a stable manner, that is, it is not necessary to consider rot difference, and further, measurement precision required in clinical examination can be satisfied.

The dry multilayered film preferably has a porous membrane adhered thereto. Examples of the porous membrane include cellulose type porous membranes such as a nitrocellulose porous membrane, a cellulose acetate porous membrane, a cellulose propionate porous membrane and a regenerated cellulose porous membrane, polysulfone porous membranes, polyether sulfone porous membranes, polypropylene porous membranes, polyethylene porous membranes and polyvinylidene chloride porous membranes. Of those, polysulfone porous membranes and polyether sulfone porous membranes are preferable.

A method of adhering the porous membrane to the dry multilayered film is not particularly limited. For example, the porous membrane can be adhered to the dry multilayered film by wetting the dry multilayered film using water in an amount of from 15 to 30 g per 1 m² of the dry multilayered film, and press bonding the porous membrane to the dry multilayered film by applying pressure of from 3 to 5 kg/cm² to the membrane at room temperature.

Further, it is preferable that fine particles of 100 µm or less are adhered to the dry multilayered film, and such a film is used as the reagent layer. Examples of the fine particles include inorganic fine particles as represented by metal oxides such as silica, alumina, zirconia and titania, and organic polymer fine particles as represented by polystyrene (PS) and polymethyl methacrylate (PMMA). Silica and polystyrene are more preferable.

A method of adhering the fine particles to the dry multilayered film is not particularly limited. For example, a method of applying an aqueous solution prepared by adding polyvinyl pyrrolidone (PVP), polyisopropyl acrylamide or a mixture of those in an amount of from 1 to 10% based on the mass of the fine particles, to the film, and then drying the resulting coating.

When the kind of the analyte is blood as in the invention, filtration is conducted using the filter for separating blood cells of the invention before supplying an analyte to the part supporting the development reaction reagent. The filtration may be conducted by combining other conventional methods. By this filtration, without operating a specific apparatus, red blood cells are removed from whole blood, and blood plasma can then be supplied to a reagent. As a result, time reaching detection can be shortened, which is preferable.

The porous membrane has a pore size of preferably from 0. 2 to 30 µm, more preferably from 0.3 to 8 µm, further preferably from 0. 5 to 4.5 µm, and particularly preferably from 0.5 to 3 µm.

The porosity of the porous membrane is preferable as being high. Specifically, the porosity is in a range of preferably from about 40 to about 95%, more preferably from about 50 to about 95%, and further preferably from about 70 to about 95%.

Examples of the porous membrane include the conventional polysulfone membrane, polyether sulfone membrane, fluorine-containing polymer membrane, cellulose acetate membrane and nitrocellulose membrane. Of those, the polysulfone membrane and the polyether sulfone membrane are preferable.

Further, porous membranes the surface of which having been hydrophilicized with hydrolysis, a hydrophilic polymer, a surfactant or the like can be used.

The hydrolysis, a hydrophilic polymer, a surfactant or the like applied to the hydrophilicization treatment can use methods and compounds generally used in hydrophilicization treatment.

The analyte is infused from an inlet of the multi-measuring dry analytical device. Any embodiment can be used so far as an analyte can be infused. For example, the flow passage may directly be connected to the outside of the multi-measuring dry analytical device.

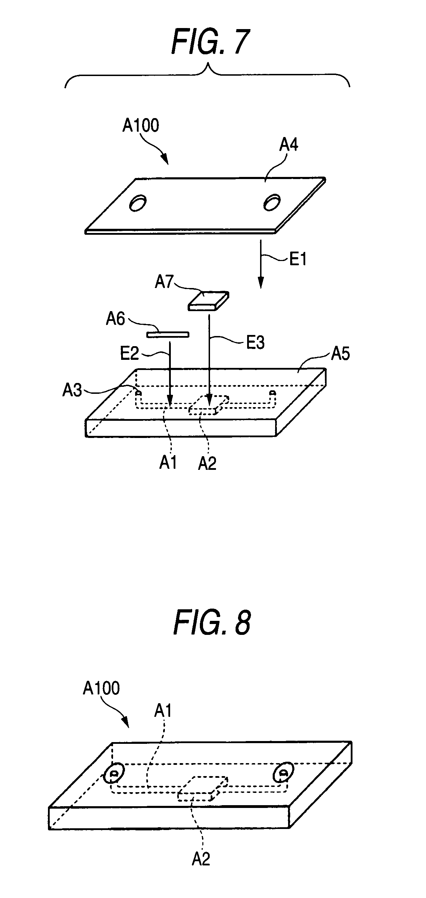

One example of the preferable embodiment of the multi-measuring dry analytical device is described below by referring to Figs. 7 and 8, but the invention is not construed as being limited thereto.

An analyte is infused from an inlet A3 of a multi-measuring dry analytical device A100. The analyte infused is derived in a part A2 supporting a development reaction reagent through a flow passage A1. As described before, a filtering member A6 for applying a filtration method is provided in the flow passage A1. In this embodiment, a method of using the filter for separating blood cells of the invention is applied to the filtration method. A development reaction reagent A7 is provided in the part A2 supporting the development reaction reagent. In Fig. 7, A1, A2 and A3 are prepared in an under material A5 using a fine processing technology. However, as described before, constitution of A1, A2 and A3 is prepared, a under lid is provided in place of the under material A5, and those may be fabricated.

A material of the multi-measuring dry analytical device can be the same materials as in the material of the flow passage, and the preferable range is also the same.

Shape and size of the multi-measuring dry analytical device may be any shape and size so far as it can be hand-carried . Specifically, the preferable shape and size are a rectangle having a side at bottom of from about 10 to 50 mm, and one having a thickness of from 2 to 10 mm.

In fabricating the rnulti-measuring dry analytical device, the same technology as the bonding technology used in connecting the part supporting the development reaction reagent and the flow passage as described before can be used.

The analyte moves in the multi-measuring dry analytical device. That is, the analyte moves from the flow passage to the part supporting the development reaction reagent by a method of, for example, utilizing pressure or utilizing capillary phenomenon. It is preferable to utilize pressure, particularly, negative pressure.

The multi-measuring dry analytical device is mounted to a blood collecting instrument to form a blood collecting unit. The blood collecting unit is described below.

The blood collecting unit is produced by mounting the multi-measuring dry analytical device to the blood collecting instrument and combining slidably while maintaining a nearly air-tight state, and this enables a closed space to form the inside pressure-reducibly. The blood collecting unit may have any shape and size so far as the multi-measuring dry analytical device can be mounted to the blood collecting instrument, can be combined slidably while maintaining a nearly airtight state, and can form a closed space pressure-reducibly in the inside.

It is preferable that the blood collecting unit is easily hand-carried and is easy to operate.

The blood collecting unit enables that by forming a closed space pressure-reducibly in the inside, the whole blood collected enters in the flow passage of the multi-measuring dry analytical device, and can be quickly lead to the part supporting the development reaction reagent.

Material of the blood collecting unit can be the same material as the material in the flow passage.

In fabricating the blood collecting unit, the same technology as in the bonding technology used in connecting the part supporting the development reaction reagent and the flow passage as described before can be used.

A blood collecting instrument in the blood collecting unit preferably has a puncture needle having a diameter of 100 µm or less and a tip angle of 20° or less. This requirement can perform puncture smoothly, and reduce pain at the time of blood collecting, which is preferable.

A bonding method of the blood collecting unit and the puncture needle can use the same technology as in the bonding technology used in connecting the part supporting the development reaction reagent and the flow passage.

The puncture needle is a hollow needle, and collects blood from a blood vessel. The whole blood is introduced into the flow passage of the multi-measuring dry analytical device by reduced pressure by sliding the blood collecting unit. The puncture needle may be, for example, the general injection needle so far as it is satisfied with the above requirement. A small-sized needle may be used from the point of blood collecting in a slight amount. Further, it is preferable to make the tip thin, thereby reducing pain at the time of blood collecting. The needle can further be prepared utilizing the above-described fine processing technology.

Material constituting the puncture needle is generally metals, and examples thereof include materials used as an injection needle, such as stainless steels, nickel-titanium alloys and tungstens. Materials preferable as metals are metals described in JIS T-3209 or JIS T-3220, and more preferably SUS 304. Further, it is possible to use resins such as the plastics as described before as the materials constituting the multi-measuring dry analytical device. Specifically, examples of the resins include PCO, PS, PC, PMMA, PE, PET, PP and PDMS.

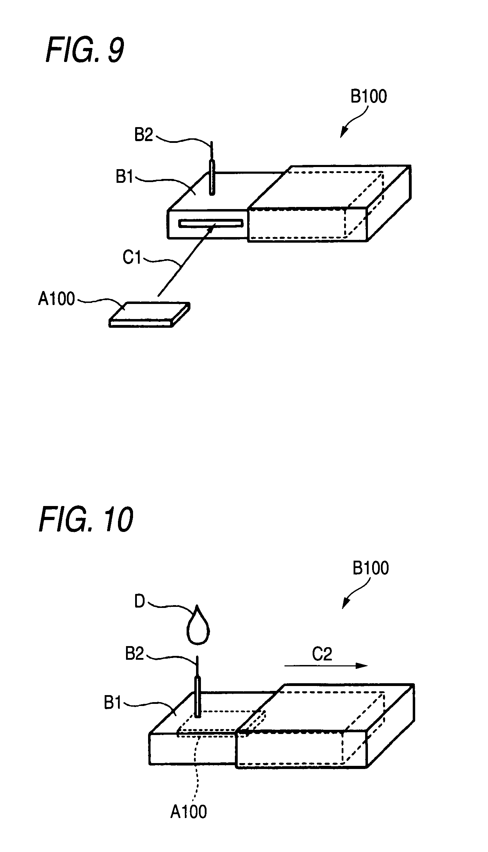

One example of the preferable embodiment of the blood collecting unit is described by referring to Figs. 9 and 10, but the invention is not construed as being limited thereto.

The multi-measuring dry analytical device A100 is mounted to a blood collecting instrument B1 from a direction of C1, thereby forming a blood collecting unit B100. After mounting, a puncture needle B2 is stuck into human being or animals to collect a whole blood D.

As described before, a part of the blood collecting instrument is slid into the direction C2, thereby reducing pressure in the inside, and the whole blood D collected is entered in a flow passage A1 of the multi-measuring dry analytical device A100 and then driven in a part A2 supporting a development reaction reagent to conduct reaction. After the reaction, the multi-measuring dry analytical device A100 is detached from the blood collecting instrument B1, and then can be provided to detection. The multi-measuring dry analytical device A100 may have either of the embodiment of detaching the same from the blood collecting instrument B1 to the other side of the blood collecting instrument B1 in the direction C1, i.e. , in the same direction when mounting, and the embodiment of detaching the same from a direction reverse to the direction C1, i.e., from the same side when mounting.

In the case that fingertip, elbow, heel or the like is cut with a lancet to collect peripheral blood, and the peripheral blood is used for test, the blood collecting instrument of the blood collecting unit does not require a puncture needle. A structure is sufficient if it has a hollow structure and a function that can lead blood to an analytical element.

### Measuring Apparatus

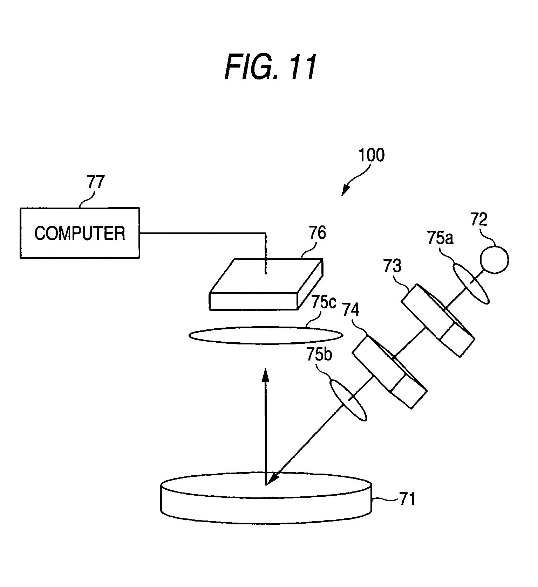

Outline structure of a measuring apparatus in the case of using the area sensor is described below by referring to Fig. 11.

A measuring apparatus 100 comprises a multi-measuring dry analytical device installation part 71 which sets an analyte to be measured; a light source 72 using a light emitting device such as a halogen lamp for emitting light to an analyte; a light-variable part 73 that changes intensity of light emitted from the light source 72; a wavelength-variable part 74 that changes wavelength of light emitted from the light source 72; lenses 75a and 75 b that parallel and condense light emitted from the light source 72; a lens 75c that condenses a reflected light from the analyte; an area sensor 76 as a light-receiving device that receives the reflected light condensed by the lens 75c; and a computer 77 that controls each part and outputs to a display or the like by obtaining measurement results according to the state of the light-variable part 73 and light intensity of light received with the area sensor 76. This embodiment has the constitution that the computer 77 controls each part, but a computer overseeing and controlling each part may be provided separately.

The multi-measuring dry analytical device is provided to the multi-measuring dry analytical device installation part 71. The part which is actually subjected to the measurement is a part supporting a development reaction reagent contacted with a filtrate (blood plasma) passed through a glass fiber filter and reacted therewith (a dry analytical element, hereinafter referred to as a "reagent-supporting part").

The light-variable part 73 functions such that a plate material of a metal mesh such as stainless steel with holes and a natural density filter (ND filter) are mechanically taken in and out between the light source 72 and the analyte, thereby changing intensity of light irradiating the analyte from the light source 72. In the initial setting, the natural density filter is in a state of being inserted between the light source 72 and the analyte. The metal mesh used hereinafter is a stainless steel mesh. A plate material of a stainless steel mesh with holes and a natural density filter (ND filter) may be set so as to be taken in and out manually.

The wavelength-variable part 74 functions such that any one of plural kinds of interference filters is mechanically taken in and out between the light source 72 and the analyte, thereby changing wavelength of light irradiating the analyte from the light source 72.

In this embodiment, the wavelength-variable part 74 is provided between the light-variable part 73 and the multi-measuring dry analytical device installation part 71, but may be provided between the light source 72 and the light-variable part 73. Further, plural kinds of interference filters may be set so as to be taken in and out manually.

The area sensor is a solid photographic device such as CCD, and functions to receive light reflected by light emitted from the light source 72 when a reagent of the reagent-supporting part in the multi-measuring dry analytical device installed in the multi-measuring dry analytical device installation part 71 reacts with an analyte such as blood, converts the received light into an electrical signal, and outputs the signal to the computer 77. The area sensor 76 can receive light reflected from the reagent-supporting part in a face unit. This makes it possible to simultaneously measure an area of each reagent, i.e., multiple items.

The computer 77 functions to convert an electrical signal according to the amount of light received output from the area sensor 76 into an optical concentration value based on the data of a calibration curve memorized in a memory or the like previously incorporated, obtain the content and the like of various components contained in the analyte from the optical concentration value, and output the same to a display or the like. When measuring multiple items, the computer 77 extracts the electrical signal according to the amount of light received output from the area sensor 76 for each area of the reagent-supporting part, and obtains the content of components contained in the analyte for each area. The computer 77 further functions to control the light-variable part 73 and the wavelength-variable part 74 according to the amount of light reflected from the analyte having light received in the area sensor 76 and the kind of the reagent to be reacted with the analyte, thereby changing the amount of light from the light source 72, or changing its wavelength.

In the measuring apparatus 100 having the above constitution, where the amount of light reflected from the analyte is small as not entering in a dynamic range of the area sensor 76, the light-variable part 73 increases intensity of light emitted from the light source 72 by removing a plate material of a stainless steel mesh or ND filter from between the light source 72 and the analyte. By this, the amount of light reflected from the analyte increases, making it possible to enter the amount of light reflected in the dynamic range of the area sensor 76. As a result, even thought the dynamic range of the area sensor 76 is narrow, the area sensor can receive the reflected light with good precision, thereby improving measurement precision of the content of components contained in the analyte.

For example, in the case of using the reagent-supporting part containing four kinds (A, B, C and D) of reagents, in the measuring apparatus 100, the amount of light reflected from each area containing the reagents A to D is obtained, and where any one of the amount of light reflected does not enter in the dynamic range of the area sensor 76, the light-variable part 73 conducts insertion and taking out of a plate material of stainless steel mesh or ND filter at constant intervals. Further, because the wavelength of light reflected from each area differs respectively, the wavelength-variable part 74 changes a plurality of interference filters in conformity with its wavelength.

For example, the embodiment that the amount of light reflected from the area containing A and B is too small as not entering in the dynamic range of the area sensor 76, the amount of light reflected from the area containing C and D enters in the dynamic range of the area sensor 76, and wavelength of light emitted when the reagents A to D react with a blood differs respectively is described below.

In the measuring apparatus 100 in this embodiment, the light source 72 exposes the reagent-supporting part to light, the area sensor receives light reflected from each area of a slide, and it is judged by the computer 77 as to whether the amount of light reflected from each area enters in the dynamic range of the area sensor 76. In this embodiment, the amount of light reflected from the area containing A and B is small as not entering in the dynamic range of the area sensor 76. Therefore, after light is emitted from the light source 72 for a given time, the computer 7 controls the light-variable part 73, making ND filter remove from between the light source 73 and the analyte. After irradiation with light for a given time in this state, the computer 7 controls the light-variable part 73, thereby making ND filter insert between the light source 72 and the analyte. By repeating this operation, plural kinds of measuring components can be measured with one multi-measuring dry analytical device with good precision.

The computer 7 controls the light-variable part 73, and also controls the wavelength-variable part 74 according to the kind of the reagents A to D, making four kinds of interference filters change in turn. The wavelength-variable part 74 acts to alternatively change a interference filter corresponding to the reagent A and a filter corresponding to the reagent B while the light-variable part 73 detaches ND filter, and acts to alternatively change a interference filter corresponding to the reagent C and a filter corresponding to the reagent D while the light-variable part 73 inserts ND filter. By this action, the content of plural kinds of measuring components contained in the analyte can be measured with one multi-measuring dry analytical device even in the case that wavelength of light emitted from plural kinds of components contained in the analyte differs respectively.

The measuring apparatus 100 can conduct the measurement with high precision even in CCD having narrow dynamic range by changing intensity of light from the light source 72. However, similar to the above, the high precision measurement is possible by not changing intensity of light, and changing exposure time (light-receiving time of reflected light) in CCD by the control of the computer 77.

In this embodiment, the analyte is irradiated with light from the light source 72, and the content of components contained in the analyte is measured from its reflected light. However, the content of components contained in the analyte may be obtained from transmitted light passed through the analyte.

In this embodiment, light reflected from the analyte is received using the area sensor such as CCD, but a line sensor may be used, without being limited to the area sensor.

CCD used in this embodiment is preferably a so-called honeycomb CCD in which light receiving parts such as photodiode are arranged on a semiconductor substrate vertically and horizontally with given intervals, and the light receiving parts contained in the adjacent each row of the light receiving parts are arranged out of line to the direction of about 1/2 row of pitch of mutual light receiving parts in the row of the light receiving parts.

In the above description, the measuring apparatus 100 changes intensity of light in real time according to the amount of reflected light from the analyte, but the content of the measuring component may be measured with the previously set sequence according to the measuring component contained in the analyte. The operation in this embodiment is described below.

The measuring apparatus 100 initiates measurement according to the pattern of measurement item when the reagent-supporting part is set to the multi-measuring dry analytical device installation part 71 and measurement items are set. First, the computer 77 selects intensity of light utilized for measurement from intensities of plural kinds, and an analyte is irradiated with light having the selected intensity. When the area sensor 76 receives light reflected from the analyte, the computer 77 outputs the measurement result according to the amount of reflected light received by the area sensor 76 and intensity of light selected as above. By a series of those operations, it is possible to conduct measurement of measuring components contained in the analyte with good precision.

Where exposure time of CCD is changed without changing intensity of light, the measuring apparatus 100 initiates measurement according to the pattern of the measuring item when the reagent-supporting part is installed in the multi-measuring dry analytical device 71 and the measuring items are set. First, the computer 77 is allowed to irradiate an analyte with light. The area sensor 76 receives light reflected from the analyte in an exposure time selected from plural kinds of exposure times by the computer 77. Finally, the computer 77 outputs the measurement result according to the amount of reflected light received by the area sensor 76 and the selected exposure time. By a series of those operations, it is possible to conduct measurement of measuring components contained in the analyte with good precision.

The measuring apparatus 100 does not limit to the embodiment that the reagent-supporting part is irradiated with light from the light source 72 as described before and the content of a component contained in the analyte is obtained by the reflected light or transmitted light, but may obtain the content of a component contained in the analyte by detecting light such as fluorescence emitted from the reagent-supporting part when the reagent-supporting part is irradiated with light from the light source 72, and further may obtain the content of a component contained in the analyte by detecting light due to light emission such as chemical emission emitted from the reagent-supporting part by completely blocking light of the light source 72 with the light-variable part 73 or making the state that the reagent-supporting part is not completely irradiated with light by not using the light source 72.

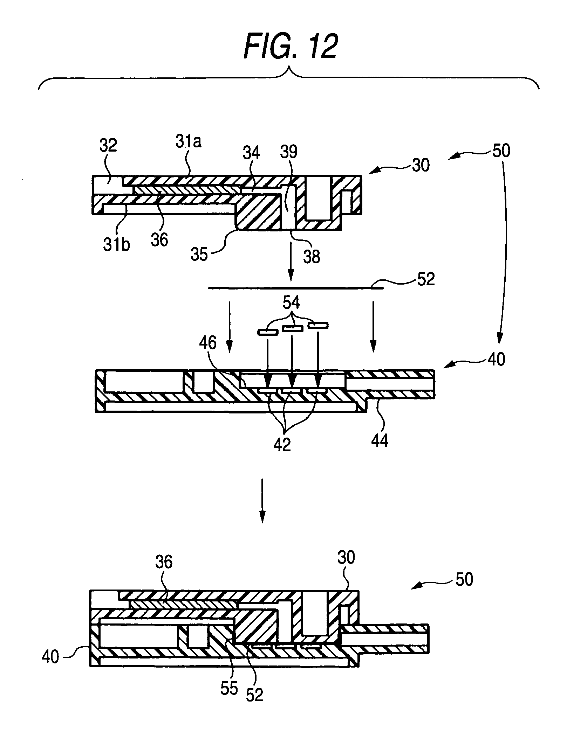

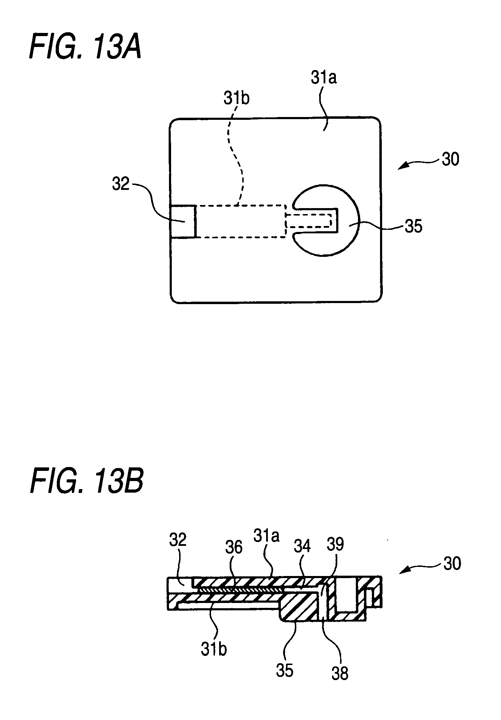

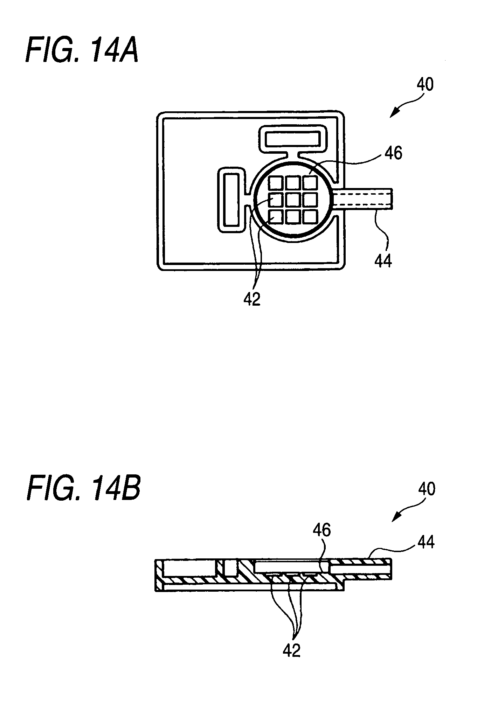

A fitting type multi-measuring dry analytical device is described below. Figs. 13A, 13B and 14A, 14B are cross sectional views showing the embodiment of the fitting type multi-measuring dry analytical device according to the invention. Figs. 13A, 13B and 14A, 14B show top views (Fig. 13A and Fig. 14A) and cross sectional views (Fig. 13B and Fig. 14B) of an upper member 30 and a lower member 40, constituting a dry analytical device 50 shown in Fig. 12, respectively.

As shown in Figs. 13A, 13B and 14A, 14B, the dry analytical device 50 according to this embodiment comprises the upper member 30 and the lower member 40, having a contour of nearly rectangular solid, and has a constitution such that the upper member 30 can be fitted to the lower member 40 thought a porous membrane 52 (Fig. 12) as a sealing member.

As shown in Figs. 13A, 13B and 14A, 14B, a supply port 32 supplying a blood is provided in the upper member 30. The supply port 32 is communicated with a flow passage 34 formed in a horizontal direction by large and small two wall plates 31a and 31b constituting the upper member 30. The flow passage 34 is filled with a filtration filter that filters a blood supplied.

A short columnar fitting projected portion 35 is formed on the undersurface side of the upper member 30, and an exhaust passage 39 communicated with the flow passage 34 is provided at the position of the columnar axis. By the exhaust passage 39, the filtrate sent by the flow passage 34 is driven downward, and sent to the lower member 40 (Figs. 14A and 14B) from an outlet 38 of the exhaust passage 39.

As shown in Figs. 13A, 13B and 14A, 14B, the lower member 40 is provided with a fitting depressed portion 46 having a bottom of a circular form, having formed thereon the fitting projected portion 35 that can be fitted.

The bottom of the fitting depressed portion 46 is provided with a cell 42 that arranges a dry analytical element 54 at, for example, 9 places in a lattice shape. The dry analytical element 54 shows a reaction such as coloration change by contacting a filtrate (blood plasma) of a blood therewith. Further, a suction nozzle 44 connecting to a suction apparatus such as suction pump (not shown) is arranged in a horizontal direction on a side wall of the fitting projected portion 46.

To form the dry analytical device 50 having those upper member 30 and lower member 40 fitted, for example, nine dry analytical elements 54 are provided in the cells 42 of the lower member 40. A porous membrane 52 having a size larger than the fitting projected portion 35 and the fitting depressed portion 46 is provided on the fitting depressed portion 46, and the fitting projected portion 35 is fitted in the fitting depressed portion 46 so as to sandwich the porous membrane 52. Thus, the dry analytical element 50 having the porous membrane 52 sandwiched in a fitting portion 55 is formed.

To analyze a blood using the dry analytical device 50, the upper member 30 and the lower member 40 are fitted as described above, and a suction pump (not shown) is connected to the suction nozzle. A blood to be used as an analyte is supplied from the supply port 32, and the suction pump is operated, thereby subjecting the blood to filtration under reduced pressure. The filtrate (blood plasma) is contacted with the dry analytical element 54 facing the outlet 38 of the exhaust passage 39, and analysis is conducted by observing coloration change or the like of the dry analytical element 54. Filtration under reduced pressure in the dry analytical device 50 may use a syringe or the like in place of a suction pump.

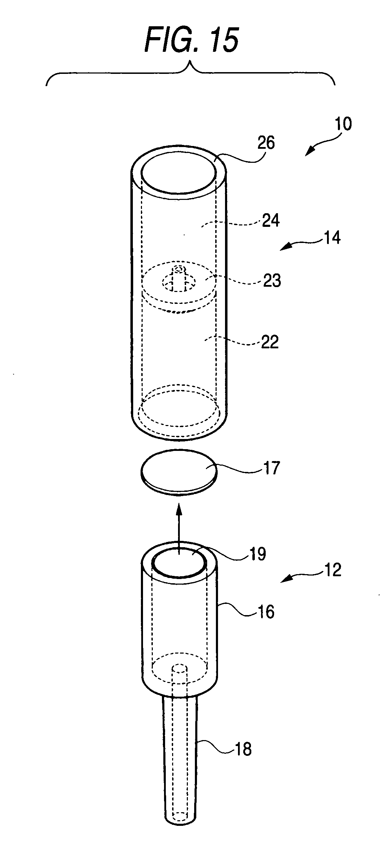

According to such a fitting type dry analytical device 50, similar to the flood filtration instrument 10 (Fig. 15) described above, bonding force between the upper member 30 and the lower member 40 can be increased by reducing pressure even by fitting the upper member 30 and the lower member 40, and sufficient air tightness and water tightness can be maintained. Therefore, a step of, for example, bonding those members can be omitted, and an expensive dry analytical device 50 having a simplified structure is obtained. This is very useful for disposable use.

The porous membrane 52 is not always required in order to maintain air tightness and water tightness necessary for filtration, similar to the blood filtration instrument 10 described above. However, bonding force in a fitting portion 55 and filtration performance can be improved by providing the porous membrane in the fitting portion 55 of the upper member 30 and the lower member 40. Therefore, it is preferable that the porous membrane 52 is present in the fitting portion 55 of the upper member 30 and the lower member 40.

### [Example]

The invention is described by referring to the following Examples, but the invention is not construed as being limited thereto.

### Example 1: Preparation of blood cell separation filter using resin-made micropillars and result of blood filtration

### (A) Preparation of resin-made micropillars

Resin-made micropillars having an equivalent circle diameter of 4 µm or less and being fixed to a substrate with contact points were prepared by the following procedures.

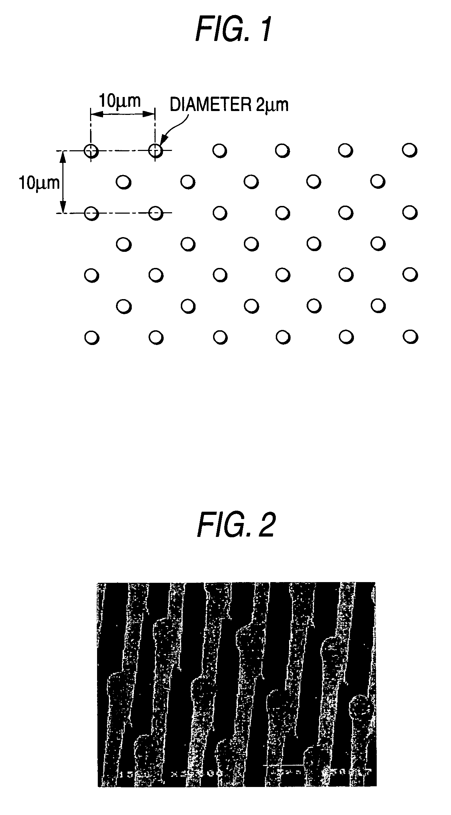

A silicon wafer was dry etched using a semiconductor processing technology to form wells each having a diameter of 3 µm and a depth of 20 µm at a distance of pitch 10 µm in a checkered pattern over an area having a width of 10 mm and a length of 10 mm. Thus, a mold for use in imprinting was prepared. Using this mold made of a silicon wafer, heat and pressure were applied to a plate of a polystyrene (PS) resin to prepare resin-made micropillars by an imprinting method. The micropillars prepared had a diameter of 2 µm and a height of 27 µm and were arranged in a checkered pattern at a distance of pitch 10 µm. The region on which the pillars could be prepared was a range having a width of 7 mm and a length of 7 mm (Figs. 1 and 2).

### (B) Preparation of blood cell separation filter using micropillars and result of blood filtration

To improve affinity for a blood, the thus-prepared PS-made micropillars were previously subjected to plasma treatment to hydrophilicize the surface thereof, the micropillars were fixed on a large-sized glass slide having a length of 52 mm and a width of 76 mm with a double-sided tape so as to face the projections upward. The micropillars were covered with a polydimethylsiloxane (PDMS) resin sheet having a thickness of about 2 mm to contact those closely, and a lid was placed thereto. The PDMS sheet previously had two holes having a diameter of about 2 mm at a distance of pitch about 7 mm, and was closely contacted such that the holes are located on both edges of the region of the micropillars on the PS-made micropillar resin. Thus, a blood cell separation filter comprising the micropillar resin and PDMS resin was prepared.

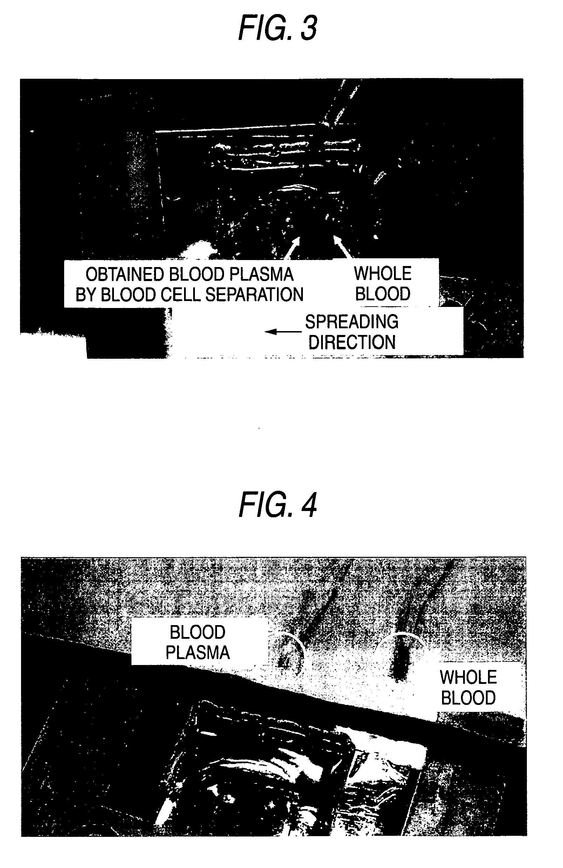

2 µl of whole blood was added dropwise to one hole of PDMS of the filter, and it was observed that the whole blood passed through the pillar region to spread toward other hole. A component not having a reddish tint spread earlier than a red component of the whole blood, and after about 2 minutes, the component not having reddish tint reached other hole. A fine paper string was prepared using kim wipe, a product of Crecia Co., and was inserted in both holes, and color tint of the blood component in the hole was visually observed. As a result, a tip of the paper string inserted in the hole to which the whole blood was added dropwise was reddish, whereas a tip of the paper string inserted in the other hole was slightly yellow, and did no show a reddish color at al. Therefore, it was understood that a filter prepared using micropillars can achieve blood cell separation, and could be functioned as a blood cell separation filter (Figs. 3 and 4).

### Example 2: Capture of red blood cell using silicon-made micropillars

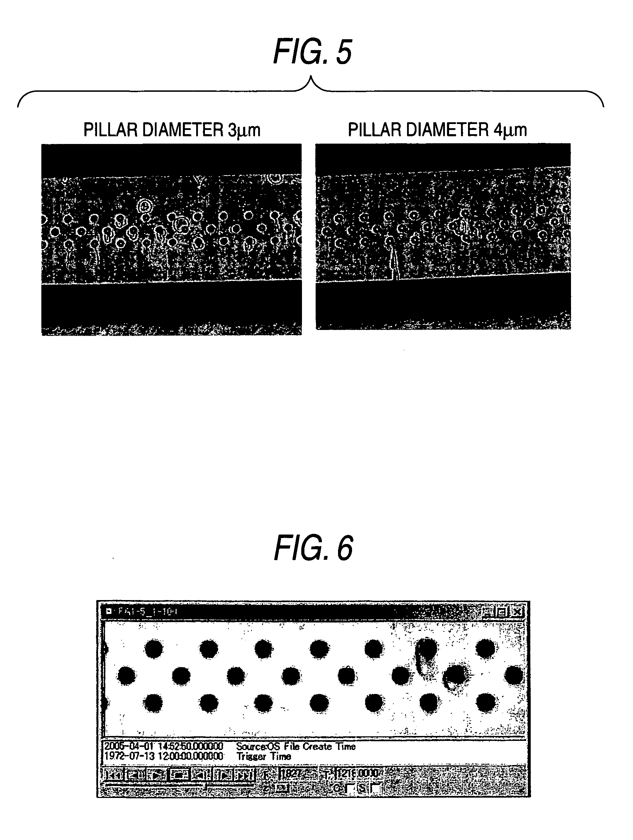

Using an apparatus MC-FAN (a product of Hitachi Haramachi Electronics Co., Ltd.) that measures by observing flow of a blood, a shape of a custom chip used for observation of MC-FAN was modified as application of conducting observation of dynamic form when cells such as red blood cells pass through a fine flow passage, thereby preparing a custom chip having provided columnar projections generally called pillars in the flow passage of the chip. Specifically, a silicon wafer was used as a substrate, the silicon wafer was dry etched using a semiconductor processing technology, and the surface of the silicon wafer was further oxidized. Pillars were prepared to have a constant height of 3.5 µm, and a diameter of 3 µm and 4 µm. The pillars were arranged with three lines at a constant pitch of 20 µm toward a direction flowing a blood. Whole blood of a healthy man collected using a heparin lithium blood collecting tube was flown in a pillar arrangement type custom chip thus prepared, and it was observed as to whether red blood cells are captured. The results obtained are shown in Fig. 5. When the pillar has a diameter of 3 µm, plural red blood cells captured by pillars could frequently be observed in a blood flowing smoothly. When the pillar has a diameter of 4 µm, red blood cells slightly captured by the pillars could be observed in a blood flowing smoothly. From those facts, it was understood that red blood cells are captured when the pillar has a diameter of 4 µm or less. It was clear that when the diameter of the pillar is 4 µm or less, red blood cells can be captured in an entangled state by using a water-insoluble substance such as a silicon-made pillar, even though not fibers.

A camera used in MC-FAN apparatus is a video camera Model LCL-211H, a product of WATEC AMERICA CORP., USA, and is a camera imaging 30 frames for 1 second. Therefore, this camera cannot confirm whether red blood cells are truly captured by fibers, pillars or the like. Therefore, the camera was placed with a high speed camera, MotionPro, a product of Nippon Roper Co., Ltd., and the same observation was conducted in the same manner as in the above. Shutter speed was 1/100,000 second. The results obtained when using pillars having a diameter of 3 µm are shown in Fig. 6. It was seen that red blood cell are captured with two pillars in an entangled state at the right side on the figure of the photograph. It could therefore be clearly indicated that red blood cells are captured with pillars.

### Example 3: Preparation of flat plate chip (fitting type dry elemental device) and its result

### (A) Preparation of flat plate chip (fitting type)

The dry analytical device 50 by fitting the upper member 30 and the lower member 40 shown in Fig. 12 through the porous membrane 52 was prepared according to the following procedures.

The upper member 30 and the lower member 40, each having a size of about 24 mm × 28 mm, molded using a transparent polystyrene were provided. Diameter of the fitting projected portion 35 and the fitting depressed portion 46 was about 9 mm.

Glass fibers obtained by previously treating a glass filter paper (GF/D, a product of Whatman Co.) for red blood cell capturing and blood plasma extraction and then being subjected to PMEA treatment, as a filtration filter 36 (analytical element) were tightly fixed to the flow passage 34 using a double-sided tape, and filled therein. Length of the glass fibers was apparently longer that an equivalent circular diameter, and further, the thickness of the glass fiber filter filled, i.e., length for convenience of glass fibers when filled, was apparently longer than an equivalent circular diameter.

Fuji Dry Chem Mount Slides GLU-P and TBIL-P (products of Fuji Photo Film Co., Ltd.), each being cut in a width of less that 2 mm and a length of less than 2 mm, were provided as the dry analytical element 54, and were arranged in cells 42 at 9 places on the lower member 40. Of the cells 42 of 9 places, the total five GLU-P were mounted at the positions of the centers and four corners, and the total four TBIL-P were mounted on places other than those places.

A polysulfone porous film (a product of fuji Photo Film Co., Ltd.) cut into a square having one side of about 18 mm was provided as the porous membrane 52. This polysulfone porous membrane was softly placed on the upper of the fitting depressed portion 46, and the upper member 30 and the lower member 40 were fitted so as to sandwich the membrane at the fitting portion between the fitting projected portion 35 and the fitting depressed portion 46 (flat plate chip).

### (B) Measuring apparatus

The measuring apparatus 100 as shown in Fig. 11 was provided. Setting of each member is as follows.

### Measuring apparatus 100: Inverted stereomicroscope

Magnification at the CCD light receiving part is the following two magnifications.

0.33 time: 33 µm/pixel at CCD part

1 time: 10 µm/pixel at CCD part

Light source 72: Luminar Ace LA-150UX, a product of Hayashi Watch Works Co., Ltd.

Wavelength-variable part 74 (interference filter): Monochrome at 625 nm, 540 nm and 505 nm, respectively

Light-variable part 73 (natural density filter): Glass filter ND-25, a product of Hoya Corporation, and homemade filter obtained by forming holes in a stainless steel plate

Area sensor 76 (CCD) : 8 bit white and black cameral module XC-7500, a product of Sony Corporation

Computer 77 (data processing (image processing)); image processor LUZEX-SE, a product of Nireco Corporation

Means for correcting reflected optical density: Following six standard plates (ceramic specification), products of Fujifilm Techno Products Co., Ltd., were provided.

Standard density plates:
A00 (reflected optical density: less than 0.5)
A05 (reflected optical density: 0.5)
A10 (reflected optical density: 1.0)
A15 (reflected optical density: 1.5)
A20 (reflected optical density: 2.0)
A30 (reflected optical density: 3.0)

### Analysis by plat plate chip

200 µl of whole blood by plain collection was injected into a supply port 32 of the fitting type dry analytical device 50 (flat plate chip) prepared above. After allowing to stand for from 10 to 20 seconds, the whole blood was spread on a glass fiber filter (filtration filter 36). A silicon-made tube was connected to a suction nozzle 44, a disposable syringe (a product of Terumo Corporation) was placed on the tip of the tube, and a piston of the syringe was softly pulled to suck.

Blood plasma extracted by filtration was passed through a polysulfone porous membrane, and added dropwise to Dry Chem Mount Slides, and GLU-P and TBIL-P slides gradually started coloration.

Time required from the injection of whole blood to the extraction of blood plasma and then the dropwise addition to the mount slide was 30 seconds.

The state of coloration of GLU-P and TBIL-P slides was imaged using the measuring apparatus 100 shown in Fig. 11 and simultaneously with CCD camera. The image obtained using LUZEX-SE was processed to obtain an average light receiving amount around the center of the images of GLU-P slide located at the center of the cell 42 and TBIL-P slide located adjacent the suction nozzle 44. The amount was converted into an optical density to obtain glucose and total bilirubin concentrations in the analyte.

When the image taken with CCD camera was processed with LUZEX-SE, each light receiving amount in a range of 1.4 mm (vertical) x 1.4 mm (horizontal) at the central portion of the images of GLU-P and TBIL-P was calculated by image processing. In this case, optical magnification 0.33 time was used. Therefore, the pixel was measured with 42 pixels (vertical) x 42 pixels (horizontal), i.e., the number of pixels of 1,764. To compare as to whether the result measured using CCD camera is correct, glucose and total bilirubin concentrations in the analyte were obtained using an automatic clinical test apparatus 7170, a product of Hitachi, Ltd. The results obtained above are shown in Table 1. In this case, because measurement wavelength differs between GLU-P slide and TBIL-P slide, the measurement was made by successively changing the wavelength of the interference filter every 5 seconds as shown in Table 2.

**TABLE 1**

| Component value in whole blood quantified with CCD detection | | |
|---|---|---|
| | Value obtained by CCD detection (mg/dl) | Measurement value by Hitachi 7170 (mq/dl) |
| Glucose | 95 | 99 |
| Total bilirubin | 0.48 | 0.44 |

**TABLE 2**

| Irradiation sequence by successively changing wavelength and amount of light | |
|---|---|
| Order | Wavelength (nm) |
| 1 | 505 |
| 2 | 540 |

| | |
|---|---|
| Order: Successively changing in the order of 1→2→1→2→1→... | |

From the above, it was seen that the dry analytical device 50 can quickly conduct measurement in a simple operation without leaking red blood cells. This was the same result as in the case of bonding the upper member 30 and the lower member 40 by ultrasonic fusion. Therefore, it was clarified that blood can be filtered and analyzed by the fitting type dry analytical device of the invention.

A dry chemistry reagent for two items was used as the dry analytical element 54 in the above-described embodiment, but the number of items can appropriately be added.

According to the invention, there is provided a filter integrated with a substrate, that is easily filled in a microstructural portion such as a microchip.

Further, according to the invention, blood cells can efficiently be captured by the filter without clogging of the blood cells, and without passing through the blood cells by failure of capturing the same. Therefore, there is provided a blood filtering instrument that can recover by filtration, blood plasma of the same component as that by centrifugal separation in a short period of time.

Furthermore, according to the invention, there is provided a blood analytical device wherein because concentration of components in blood plasma does not change, test accuracy can further be increased; and further, it is safe, operation is simple and many items can quickly be subjected to operations up to detection, in a form to be easily incorporated.

The entire disclosure of each and every foreign patent application from which the benefit of foreign priority has been claimed in the present application is incorporated herein by reference, as if fully set forth.

## Claims

1. A filter for separating blood cells, which comprises:
a substrate; and
at least one water-insoluble substance fixed to the substrate, wherein the at least one water-insoluble substance has an equivalent circle diameter of 4 µm or less and a height equal to or larger than the equivalent circle diameter,
wherein blood cells are separated by substantially being captured with the at least one water-insoluble substance.

2. The filter for separating blood cells according to claim 1,
wherein the at least one water-insoluble substance comprises a first water-insoluble substance and a second water-insoluble substance, and
a distance between the first and second water-insoluble substances is 4 µm or more.

3. The filter for separating blood cells according to claim 1,
wherein the at least one water-insoluble substance is a plurality of water-insoluble substances, and
the plurality of water-insoluble substances are arranged in a plurality of rows at even intervals in each row.

4. The filter for separating blood cells according to claim 3,
wherein the interval in each row is 4 µm or more.

5. The filter for separating blood cells according to claim 3,
wherein the plurality of water-insoluble substances are arranged in a checkered pattern.

6. The filter for separating blood cells according to claim 3,
wherein the plurality of water-insoluble substances are pillars.

7. The filter for separating blood cells according to claim 1, which has an inlet portion and an outlet portion,
wherein the at least one water-insoluble substance is a plurality of water-insoluble substances, and
the plurality of water-insoluble substances are arranged from the inlet portion to the outlet portion in a plurality of rows at even intervals in each row, and
wherein an interval in the inlet portion and an interval in the outlet portion are substantially 1.5 times or more different.

8. A blood filtering instrument, which comprises a filter for separating blood cells according to claim 1.

9. A method for separating blood cells, which comprises utilizing a filter for separating blood cells according to claim 1.

10. A blood pre-treatment element, which comprises a filter for separating blood cells according to claim 1.

11. An analytical element, which comprises a filter for separating blood cells according to claim 1.
